(19) **Europäisches Patentamt / European Patent Office / Office européen des brevets**

(11) **EP 4 289 345 A1**

(12) **EUROPEAN PATENT APPLICATION**
published in accordance with Art. 153(4) EPC

(43) Date of publication:
**13.12.2023  Bulletin 2023/50**

(21) Application number: **22749882.1**

(22) Date of filing: **13.01.2022**

(51) International Patent Classification (IPC):
*A61B 5/00* (2006.01)    *H01L 31/0328* (2006.01)
*H01L 31/18* (2006.01)    *H01L 31/028* (2006.01)
*H01L 31/032* (2006.01)

(52) Cooperative Patent Classification (CPC):
**A61B 5/00; H01L 31/028; H01L 31/032;**
**H01L 31/0328; H01L 31/18**

(86) International application number:
**PCT/KR2022/000611**

(87) International publication number:
**WO 2022/169128 (11.08.2022 Gazette 2022/32)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **04.02.2021  KR 20210015959**

(71) Applicant: **Ajou University Industry-Academic**
**Cooperation**
**Foundation**
**Gyeonggi-do 16499 (KR)**

(72) Inventors:
• **SEO, Hyung Tak**
 **Seoul 06544 (KR)**
• **KUMAR, Mohit**
 **Suwon-si Gyeonggi-do 16499 (KR)**

(74) Representative: **Laine IP Oy**
**Porkkalankatu 24**
**00180 Helsinki (FI)**

(54) **BIO-SENSING DEVICE COMPRISING PHOTOELECTRIC ELEMENT**

(57)    A bio-sensing device having a photoelectric element is disclosed. The bio-sensing device includes an infrared pulse generator configured to irradiate infrared pulsed light to a target; the photoelectric element configured to receive the infrared pulsed light which has transmitted through the target, and to generate photocurrent based on the received light; and a sensing element configured to measure a magnitude of either a first peak current of the photocurrent corresponding to a leading edge of the infrared pulsed light or a second peak current of the photocurrent corresponding to a trailing edge of the infrared pulsed light, and to analyze the target based on the measurement result.

FIG. 1

EP 4 289 345 A1

## Description

## FIELD

The present disclosure relates to a bio-sensing device having a photoelectric element which receives infrared pulsed light which has transmitted through a target and generates photocurrent based on the received light.

## DESCRIPTION OF RELATED ART

Effective utilization of photons to generate a detectable electrical signal has been recognized as one of the most attractive fields for centuries due to its potential to monitor damage in the human body. In particular, damage detection and imaging of the human body using near-infrared ray (NIR) has been actively studied in the field of medical applications because light damage due to the NIR is small, and the NIR reaches a deep portion of a tissue.

However, in order to apply optoelectronics for body damage detection, there are many requirements. For example, in order to acquire error-free data, development of a high-performance and energy-efficient photoelectric element is essential.

In general, a narrow band gap material such as indium, gallium, arsenic, lead, gallium, arsenic, etc. has been widely used as an active layer material for detecting the near-infrared ray wavelength.

However, this active layer material requires a very complex and precise process including doping, etching and epitaxy, thus making it difficult to produce the same on a commercial scale.

Moreover, a conventional photoelectric element has a problem in that performance thereof is extremely degraded in the near-infrared NIR range. Therefore, development of a device capable of generating an appropriate self-bias, generating sufficient photon absorption inducing carriers, separating carriers, and collecting carriers is required.

## DISCLOSURE

## TECHNICAL PURPOSE

One purpose of the present disclosure is to provide a bio-sensing device having a photoelectric element which receives infrared pulsed light which has transmitted through a target and generates photocurrent based on the received light, and thus capable of analyzing deformation, damage, etc. of the target.

Another purpose of the present disclosure is to provide a method for manufacturing the photoelectric element.

## TECHNICAL SOLUTION

A first aspect of the present disclosure provides a bio-sensing device with a photoelectric element, the device comprising: an infrared pulse generator configured to irradiate infrared pulsed light to a target; the photoelectric element configured to receive the infrared pulsed light which has transmitted through the target, and to generate photocurrent based on the received light; and a sensing element configured to measure a magnitude of either a first peak current of the photocurrent corresponding to a leading edge of the infrared pulsed light or a second peak current of the photocurrent corresponding to a trailing edge of the infrared pulsed light, and to analyze the target based on the measurement result.

In one embodiment of the device, a pulse period of the infrared pulsed light is in a range of 1 ms to 0.1 s, wherein a ratio of a pulse width to the pulse period of the infrared pulsed light is in a range of 1 to 10%.

In one embodiment of the device, the photoelectric element includes: a p-type semiconductor layer; an n-type semiconductor layer formed on the p-type semiconductor layer such that a PN junction is formed between the p-type semiconductor layer and the n-type semiconductor layer, wherein the infrared pulsed light is incident on the n-type semiconductor layer; and a transparent current collector formed on a surface of the n-type semiconductor layer.

In one embodiment of the device, the photoelectric element generates the photocurrent using a self-bias generated by the PN junction.

In one embodiment of the device, the p-type semiconductor layer includes p-type silicon (p-Si), wherein the n-type semiconductor layer includes titanium dioxide ($TiO_2$).

In one embodiment of the device, the transparent current collector includes at least one selected from silver nanowire, IGZO (Indium Gallium Zinc Oxide), IZO (Indium Zinc Oxide), SIZO (Silicon Indium Zinc Oxide), HIZO (Hafnium Indium Zinc Oxide), ZTO (Zinc Tin Oxide), ZnO, $Ga_2O_3$, $In_2O_3$, and $SnO_2$.

In one embodiment of the device, the sensing element is electrically connected to and disposed between the p-type semiconductor layer and the n-type semiconductor layer.

In one embodiment of the device, the bio-sensing device further comprises an image generator configured to generate a mapping image of the target based on change in a magnitude of the peak current measured by the sensing element.

A second aspect of the present disclosure provides a method for manufacturing a photoelectric element, the method comprising: depositing a titanium thin film on a substrate on which a p-type semiconductor layer has been formed in a sputtering process; oxidizing the titanium thin film to form a titanium dioxide ($TiO_2$) thin film; and forming a transparent current collector on a surface of the titanium dioxide ($TiO_2$) thin film.

In one embodiment of the method, the titanium dioxide ($TiO_2$) thin film is formed to have a thickness of 10 to 100 nm.

In one embodiment of the method, the p-type semiconductor layer includes p-type silicon (p-Si). In one embodiment of the method, the transparent current collector includes at least one selected from silver nanowire, IGZO (Indium Gallium Zinc Oxide), IZO (Indium Zinc Oxide), SIZO (Silicon Indium Zinc Oxide), HIZO (Hafnium Indium Zinc Oxide), ZTO (Zinc Tin Oxide), ZnO, $Ga_2O_3$, $In_2O_3$, and $SnO_2$.

## TECHNICAL EFFECT

The bio-sensing device of the present disclosure has the photoelectric element that receives the infrared pulsed light which has transmitted through the target and generates the photocurrent based on the received light. Further, the bio-sensing device of the present disclosure has the sensing element that measures the magnitude of the peak current of the generated photocurrent. Thus, the device may easily and accurately detect the damage or deformation of the target based on the analyzing result of the peak current magnitude.

Moreover, the bio-sensing device of the present disclosure may generate the mapping image of the target based on the change in the magnitude of the peak current measured by the sensing element. Thus, the operator of the device may visually detect whether or not the target is damaged, based on the generated image, and thus may analyze the target.

## BRIEF DESCRIPTION OF DRAWINGS

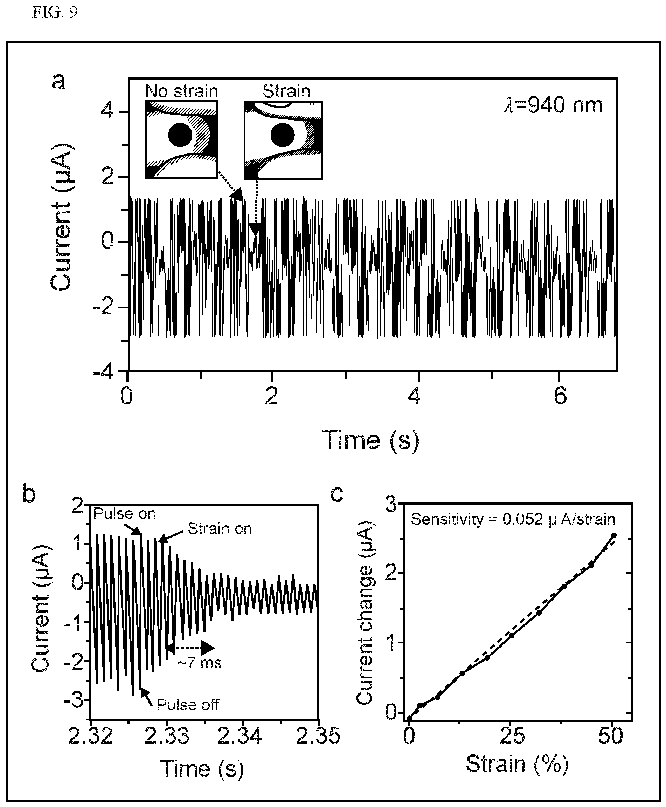

FIG. 1 shows a bio-sensing device having a photoelectric element according to an embodiment of the present disclosure.
FIG. 2 shows a photoelectric element and a sensing element according to an embodiment of the present disclosure.
FIG. 3 shows a graph of a peak current generated by the photoelectric element of the present disclosure based on pulsed light.
FIG. 4 is a schematic diagram showing a method of manufacturing a photoelectric element according to an embodiment of the present disclosure.
(a) in FIG. 5 shows results of measuring light transmittance based on a wavelength of a Ti/FTO film before and after annealing.
(b) and (c) in FIG. 5 show results of measuring a chemical composition of the Ti/FTO film using X-ray photoelectron spectroscopy (XPS).
(a) to (c) in FIG. 6 represent SEM images of a photoelectric element according to an embodiment of the present disclosure.
(d) to (g) in FIG. 6 represent EDS analysis images of a photoelectric element according to an embodiment of the present disclosure.
(a) and (b) in FIG. 7 show results of measuring photoresponse characteristics of a photoelectric element according to the present disclosure.

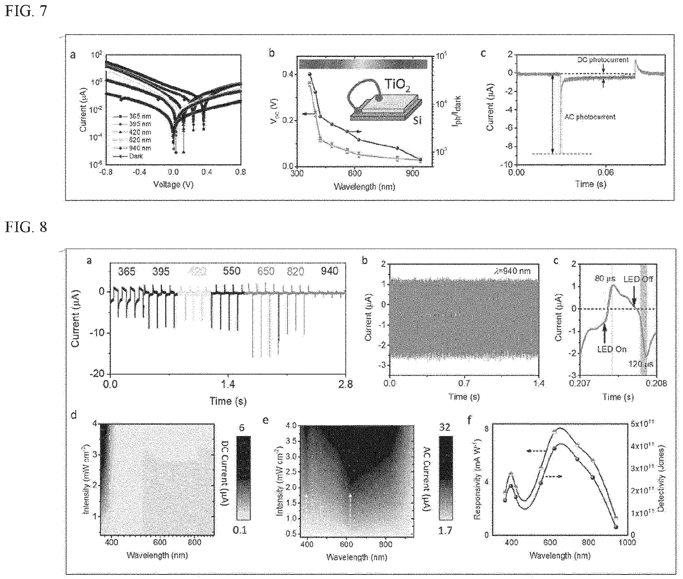

(c) in FIG. 7 shows a transient-photoresponse (I-t) result of a photoelectric element without external bias at pulsed light ($\lambda$ = 395 nm, P = 1 mW cm$^{-2}$, frequency, f = 14 Hz).
FIG. 8 shows a result of measuring broadband photoresponse characteristics of a photoelectric element according to an embodiment of the present disclosure.
(a) and (b) in FIG. 9 show results of measuring a photocurrent output under a self-biased condition with or without external strain after passing near-infrared pulsed light through a finger.
(c) in FIG. 9 is a graph showing change in photocurrent (Iac) based on applied external strain.

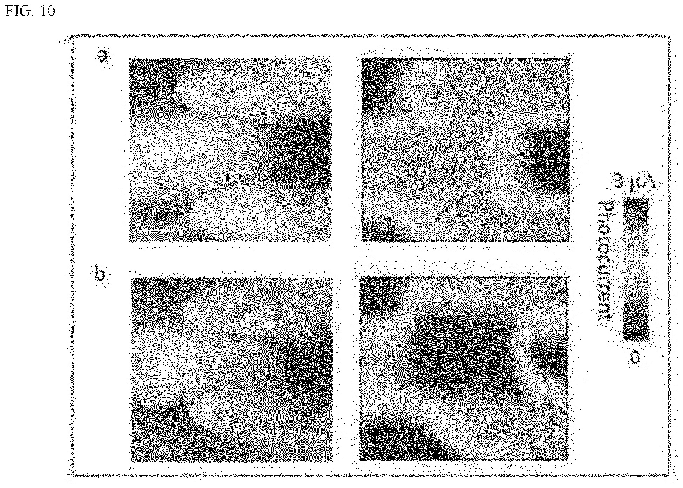

FIG. 10 is a mapping image according to external strain obtained using a bio-sensing device according to an embodiment of the present disclosure.

## DETAILED DESCRIPTIONS

Hereinafter, a gadget for measuring a retroreflected signal according to an embodiment of the present disclosure will be described in detail with reference to the accompanying drawings. The present disclosure may have various changes and may have various forms. Thus, specific embodiments are illustrated in the drawings and described in detail in the text. However, this is not intended to limit the present disclosure to the specific forms as disclosed. It should be appreciated that the present disclosure includes all modifications, equivalents, or substitutes included in the spirit and scope of the present disclosure. Like reference numerals have been used for like elements throughout the descriptions of the drawings. For simplicity and clarity of illustration, elements in the drawings are not necessarily drawn to scale.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described under could be termed a second element, component, region, layer or section, without departing from the spirit and scope of the present disclosure.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "comprising", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

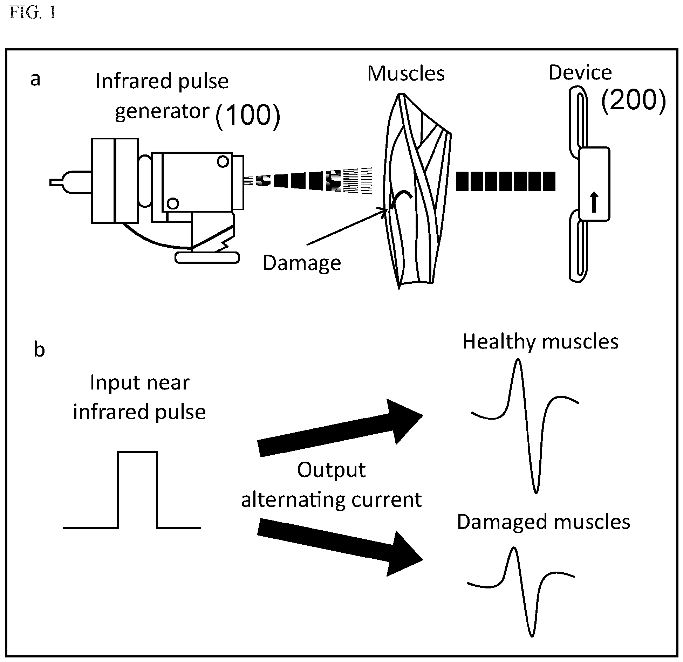

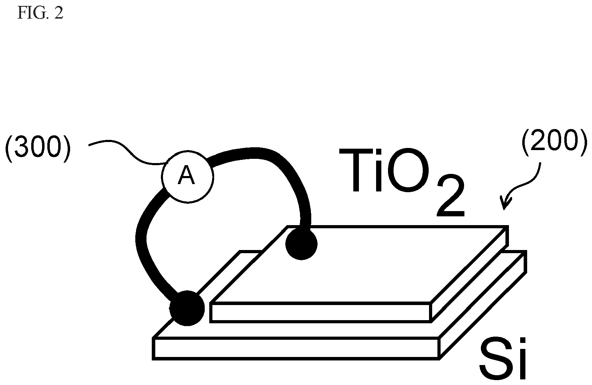

FIG. 1 shows a bio-sensing device having a photoelectric element according to an embodiment of the present disclosure, and FIG. 2 shows a photoelectric element and a sensing element according to an embodiment of the present disclosure.

Referring to FIGS. 1 and 2, the bio-sensing device of the present disclosure may include an infrared pulse generator 100, a photoelectric element 200, and a sensing element 300.

The infrared pulse generator 100 generates infrared pulsed light and irradiates a target with the infrared pulsed light. In this regard, the target may mean a biological material or a human body.

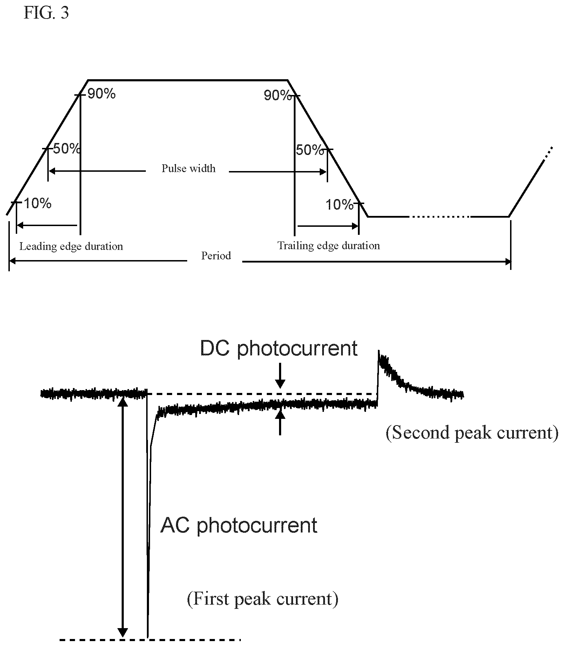

The infrared pulsed light irradiated to the target is composed of a period, a pulse width, a rising edge (leading edge), and a falling edge (trailing edge), as shown in FIG. 3, and the pulse period of the infrared pulsed light in accordance with the present disclosure may be in a range of 1 ms to 0.1 s. A ratio of the pulse width to the pulse period may be preferably in a range of 1 to 10%. However, the present disclosure is not limited thereto. Since the device may operate at a frequency of up to kHz, another operating frequency may be selected.

The photoelectric element 200 receives the infrared pulsed light which has transmitted through the target, and generates photocurrent based on the received light. The photoelectric element 200 is electrically connected to the sensing element 300.

In one embodiment, as shown in FIG. 3, the photoelectric element 200 may generate a first peak current corresponding to the leading edge of the infrared pulsed light which has transmitted through the target and a second peak current corresponding to the trailing edge thereof. In this regard, as shown in FIG. 1, a magnitude of the first peak current or the second peak current changes based on a density of the target or whether the target is damaged. Therefore, the target may be analyzed by the sensing element 300 measuring the magnitude of either the first peak current or the second peak current.

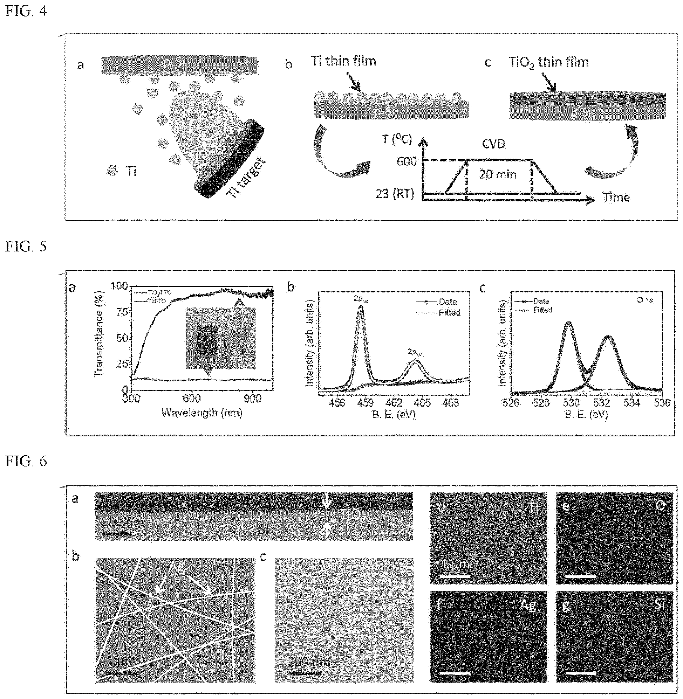

Specifically, the photoelectric element 200 may include a p-type semiconductor layer, an n-type semiconductor layer, and a transparent current collector. (See FIG. 4).

A PN junction is formed between the p-type semiconductor layer and the n-type semiconductor layer. The p-type semiconductor layer may be made of a semiconductor material doped with a p-type dopant. Preferably, the p-type semiconductor layer may include a p-type silicon (p-Si) doped with a p-type dopant. However, the present disclosure is not limited thereto.

The n-type semiconductor layer is formed on the p-type semiconductor layer such that the PN junction is formed between the p-type semiconductor layer and the n-type semiconductor layer. The infrared pulsed light is incident on the n-type semiconductor layer. In one embodiment, the n-type semiconductor layer preferably includes titanium dioxide ($TiO_2$). However, the present disclosure is not limited thereto.

The transparent current collector is formed on a surface of the n-type semiconductor layer and serves to provide a path for effective charge collection when the photocurrent is generated. Such a transparent current collector may include a silver nanowire, a transparent metal oxide conductor, and the like. For example, the transparent current collector may include at least one selected from the silver nanowire, IGZO (Indium Gallium Zinc Oxide), IZO (Indium Zinc Oxide), SIZO (Silicon Indium Zinc Oxide), HIZO (Hafnium Indium Zinc Oxide), ZTO (Zinc Tin Oxide), ZnO, $Ga_2O_3$, $In_2O_3$, and $SnO_2$. Most preferably, the silver nanowire may be used as a material of the transparent current collector.

In one embodiment, in the photoelectric element 200 of the present disclosure, when the infrared pulsed light is incident on the n-type semiconductor layer, a self-bias is generated due to the PN junction. Thus, the photocurrent may be generated by this self-bias.

The sensing element 300 may analyze the target based on a measuring result of the magnitude of either the first peak current corresponding to the leading edge or the second peak current corresponding to the trailing edge of the photocurrent.

In one embodiment, the sensing element 300 may be electrically connected to and disposed between the p-type semiconductor layer and the n-type semiconductor layer of the photoelectric element 200. The sensing element 300 may measure the magnitude of the photocurrent (the magnitude of either the first peak current or the second peak current) as generated by the self-bias generated due to the PN junction between the p-type semiconductor layer and the n-type semiconductor layer, and may analyze the target based on the measurement result.

Preferably, the target may be analyzed based on the measuring result of the magnitude of the first peak current of the photocurrent corresponding to the leading edge of the infrared pulsed light. This is because change in the magnitude of the first peak current is greater than change in the magnitude of the second peak current.

In one embodiment, the bio-sensing device of the present disclosure may further include an image generator (not shown).

The image generator generates a mapping image of the target based on the change in the magnitude of the peak current measured by the sensing element 300, such that an operator visually detects whether or not the target is damaged, based on the generated image, and thus analyzes the target.

As described above, the bio-sensing device of the present disclosure has the photoelectric element that receives the infrared pulsed light which has transmitted through the target and generates the photocurrent based on the received light. Further, the bio-sensing device of the present disclosure has the sensing element that measures the magnitude of the peak current of the generated photocurrent. Thus, the device may easily and accurately detect the damage or deformation of the target based on the analyzing result of the peak current magnitude.

Moreover, the bio-sensing device of the present disclosure may generate the mapping image of the target based on the change in the magnitude of the peak current measured by the sensing element. Thus, the operator of the device may visually detect whether or not the target is damaged, based on the generated image, and thus may analyze the target.

FIG. 4 is a schematic diagram showing a method of manufacturing a photoelectric element according to an embodiment of the present disclosure.

Referring to FIG. 4, a photoelectric element manufacturing method according to another embodiment of the present disclosure may include depositing a titanium thin film on a substrate on which a p-type semiconductor layer is formed in a sputtering process in S 110, forming a titanium dioxide ($TiO_2$) thin film by oxidizing the titanium thin film in S120, and forming a transparent current collector on a surface of the titanium dioxide ($TiO_2$) thin film in S130.

First, the step S 110 of depositing the titanium thin film on the substrate on which the p-type semiconductor layer is formed is performed in the sputtering process.

In one embodiment, the sputtering process may be preferably performed by applying RF power of 80 to 120 W at a temperature of 10 to 40°C under an argon gas flow of 25 to 35 sccm. However, the present disclosure is not limited thereto.

Moreover, the p-type semiconductor layer may be made of a semiconductor material doped with a p-type dopant. Preferably, the p-type semiconductor layer may include p-type silicon (p-Si) doped with a p-type dopant. However, the present disclosure is not limited thereto.

Next, the step S120 of forming the titanium dioxide ($TiO_2$) thin film by oxidizing the titanium thin film is performed.

In one embodiment, oxidation of the titanium thin film may be performed in a chemical vapor deposition process (CVD) at a temperature of 500 °C to 700 °C. However, the present disclosure is not limited thereto.

Moreover, the titanium dioxide ($TiO_2$) thin film is preferably formed to have a thickness of 10 to 100 nm. However, the present disclosure is not limited thereto.

Finally, the step S130 of forming the transparent current collector on the surface of the titanium dioxide ($TiO_2$) thin film may be performed.

In one embodiment, the transparent current collector may include at least one selected from the silver nanowire, IGZO (Indium Gallium Zinc Oxide), IZO (Indium Zinc Oxide), SIZO (Silicon Indium Zinc Oxide), HIZO (Hafnium Indium Zinc Oxide), ZTO (Zinc Tin Oxide), ZnO, $Ga_2O_3$, $In_2O_3$, and $SnO_2$. However, the present disclosure is not limited thereto.

According to the present disclosure, the titanium dioxide thin film may be formed by oxidizing the titanium thin film, thereby forming a smooth interface between the p-type semiconductor layer and the titanium dioxide thin film, and thus, improving the performance of the photoelectric element.

Hereinafter, the contents of the present disclosure will be additionally described along with specific examples.

<Manufacturing of photoelectric element>

Titanium thin films of various thicknesses ranging from 4 to 80 nm were respectively deposited on glass substrates on which ultrasonically cleaned p-type silicon (p-Si) (natural oxide coating) and FTO (Fluorine doped Tin Oxide) were coated using a large-area sputtering technique. At this time, the sputtering was performed at a room temperature (300 K) under an Ar flow of 30 sccm and a radio frequency (RF) power of 100 W. A distance between the substrate and a titanium target was fixed to 7.5 cm.

Next, Ti/Si and Ti/FTO samples were annealed to form metal oxides using a chemical vapor deposition (CVD) apparatus at 600°C under a controlled oxygen flow of 50 sccm. (See (a) to (c) in FIG. 4).

<Characteristics of photoelectric element>

1. In order to identify the formation of the metal oxide of the photoelectric element, the light transmittances based on the wavelength of the Ti/FTO film before and after annealing were measured, and the result is shown in (a) in FIG. 5.

As shown in (a) in FIG. 5, the film after annealing became very transparent (> 80%). The product after the oxidation of the titanium thin film did not exhibit no significant absorption in the visible light range ($\lambda$ = 400 to 700 nm). This indicated that a high-quality film was formed, and there was no in-gap active state.

Moreover, referring to the original photos of the Ti/FTO and $TiO_2$/FTO samples inset in (a) in FIG. 5, clear color change from dark black to transparent may be confirmed.

(b) and (c) in FIG. 5 show the results of measuring the chemical composition of the Ti/FTO film using the X-ray photoelectron spectroscopy (XPS).

As shown in (b) in FIG. 5, the XPS spectrum of Ti 2p includes two peaks at binding energies of 458.46 and 464.17 eV, respectively. The high-intensity peak (Ti, $2p_{3/2}$) at 458.46 eV corresponds to $Ti^{4+}$ in $TiO_2$ (458.6 to 459.2 eV), and the second peak (Ti, $2p_{1/2}$) corresponds to spinorbit coupling.

Referring to these spectra, the peak of the Ti/FTO film is present at a significantly different position from that of metal $Ti^O$ (453.7 to 454.2 eV) or $Ti^{2+}$ (454.9 to 455.2 eV). Thus, the formation of the metal oxide may be identified. Moreover, an area ratio (2.2:1) of this peak is similar to a branching ratio (2:1) of a 2p line.

In one embodiment, referring to (c) in FIG. 5, the measured O 1s peaks correspond to two Gaussian components centered at about 529.80 and 532.39 eV, respectively. The first peak at 529.8 eV is due to the presence of oxygen at the lattice site, and the second peak is related to an -OH group adsorbed on the surface. Based on the X-ray photoelectron spectroscopy result, the formation of titanium dioxide ($TiO_2$) was identified.

2. Morphological analysis of the thin film on which the metal oxide was formed was performed using a scanning electron microscope (SEM).

(a) in FIG. 6 is a cross-sectional SEM image of the thin film. It may be observed that the $TiO_2$ thin film is formed to have a thickness of about 50 nm, and no macroscopic cracks or defects may be identified.

Moreover, a smooth interface formation between the Si layer and the $TiO_2$ layer may be observed, which is desirable for efficient charge transport and consequently enhances the performance of the photoelectric element.

In one embodiment, in order to provide a path for effective charge collection, the silver nanowire (Ag-NW) was formed on the surface of the $TiO_2$ layer. A plan-view SEM image of the $TiO_2$ layer having the silver nanowire (AgNW) formed on the surface thereof is shown in (b) in FIG. 6.

Referring to (b) and (c) in FIG. 6, it may be identified that the film is composed of uniformly distributed nanostructures with lateral sizes of 30 to 60 nm, as indicated by circles.

Moreover, elemental distributions of titanium (d), oxygen (e), silver (f) and silicon (g) were identified based on energy dispersive X-ray spectroscopy (EDS), and the results are shown in (d) to (g) of FIG. 6, respectively.

Referring to (d) to (g) in FIG. 6, the elemental ratios of Ti and O are 66.2% and 33.8%, respectively, indicating that the Ti/O elemental ratio of 1.95 is similar to the ideal stoichiometric composition ratio of the $TiO_2$ layer.

3. The steady-state photoresponse property of the photoelectric element having a $TiO_2$/Si heterostructure was measured by performing current-voltage I-V measurements under dark conditions and various wavelengths of $\lambda$, = 365 to 940 nm at constant illumination (P = 4 mW cm$^{-2}$).

Referring to (a) of FIG. 7 which shows the current-voltage I-V curve, the asymmetry of the I-V curve with a current ratio of $I_{-0.8}/I_{+0.8}$ = 18 (that is, a current ratio at $\pm$0.8 V) under the dark condition may be identified, which is due to band alignment at the TiO/Si interface.

Moreover, it may be observed that the current value of the I-V curve obtained when irradiated with light of various wavelengths shift upward, compared with the I-V curve obtained under the dark condition. Thus, it may be identified that photo-induced electron-hole (e-h) pairs are generated.

In one embodiment, the wavelength-dependent shape (that is, the self-bias, 0 V) of the photocurrent under the absence of applied voltage not only may ensure broadband (ultraviolet (UV) to near-infrared (NIR)) optical activity, but also may separate the photoexcited electron-hole (e-h) pairs from each other under the action of the PN junction built-in potential ($E_b$) of the photoelectric element. This function is further demonstrated by the shift of the open circuit voltage ($V_{oc}$) (the photocurrent line away from the dark line of the voltage scale).

(b) in FIG. 7 shows the open circuit voltage (Voc) and a ratio ($I_{ph}/I_{dark}$) of photocurrent (Iph) to dark current (Idark).

The current significantly increases from about 15 pA under the dark condition to 0.42 $\mu$A under $\lambda$ = 365 nm (P = 4 mW cm$^{-2}$) light irradiation at 0 V bias. This means that the $I_{ph}/I_{dark}$ ratio is as high as $4.2 \times 10^4$, indicating that the photoelectric device of the present disclosure is applicable to a high-performance UV sensor.

4. On the other hand, the photoelectric device of the present disclosure is sensitive to a range of visible light ($\lambda$ = 400 to 700 nm) to near-infrared ($\lambda$ = 700 to 940 nm) light under a self-biased condition.

(c) in FIG. 7 shows the transient-photoresponse (I-t) result of the photoelectric device without external bias under the pulsed light ($\lambda$ = 395 nm, P = 1 mW cm$^{-2}$, frequency, f = 14 Hz).

Unlike conventional direct current (DC) photocurrent ($I_{dc}$), two peaks appeared during light irradiation and extinction, indicating that alternating current (AC) photocurrent ($I_{ac}$) was generated.

A magnitude of the photocurrent ($I_{ac}$) peak corresponding to the leading edge of the pulsed light (-8.8 $\mu$A) was significantly larger than that of the photocurrent ($I_{dc}$) peak corresponding to the trailing edge of the pulsed light (-0.48 $\mu$A) under the same illuminance. For comparison, the magnitude of the current increase was calculated as a ratio $(|I_{dc+ac}|-|I_{dc}|)/|I_{dc}|$, which was measured to be about 1733%.

The shapes of the current peaks generated during light irradiation and extinction may correspond to relative shift and rearrangement of quasi-Fermi levels of the photoelectric device having a heterostructure under periodic light irradiation.

<Broadband photoresponse characteristics of photoelectric device>

(a) in FIG. 8 shows the current-time (I-t) response spectrum of the photoelectric device at different wavelengths ($\lambda$ = 365 to 940 nm) under the same pulse condition (P = 1 mW cm$^{-2}$, 0 V, f = 14 Hz).

Referring to (a) of FIG. 8, photocurrent ($I_{ac}$) peaks were observed at all wavelengths, whereas the magnitudes of the photocurrent ($I_{dc}$ and $I_{ac}$) peaks varied, thus confirming that the photoelectric device with the PN junction structure generates the photocurrent in a wide band.

In the photoelectric device, repeatability and stability act as important factors in actual application of the photoelectric device.

(b) in FIG. 8 shows the performance of the photoelectric device after performing multiple cycles. For 3 months after the device was fabricated, reproducible response was exhibited without any passivation or capping structure, and there was no noticeable performance degradation or deviation, and excellent durability and stability were exhibited.

(c) in FIG. 8 shows a photocurrent rise time ($\tau_r$, the time required for the photocurrent to increase from 10% to 90% of the peak) and a photocurrent fall time ($\tau_f$, the time required for the photocurrent to decrease from 90% to 10% of the peak) as measured when the photoelectric device operates at high speed. Based on a result of the measurement, the photocurrent rise time and the photocurrent fall time were measured as 80 and 120 $\mu$s, respectively.

Further, a cutoff frequency ($f_{3dB}$) at 3 dB was estimated to be 4.3 kHz using a following equation: $f_{3dB}$=0.35/$\tau_r$. This response time allows the photoelectric device to operate at high data rate.

(d) and (e) in FIG. 8 are comprehensive maps of the photocurrents ($I_{dc}$ and $I_{ac}$) at various illuminations (0.4 to 4 mW cm$^{-2}$), which indicate the broadband response of the photoelectric device.

Referring to (d) and (e) of FIG. 8, the maximum $I_{ac}$ reached about 32$\mu$A at $\lambda$ = 620 nm (P = 4mW cm$^{-2}$), while the maximum $I_{dc}$ was only about 0.1$\mu$A at the same illumination intensity.

That is, under similar measurement condition, at $\lambda$ = 620 nm, $I_{ac}$ exhibits a significant photocurrent magnitude 31,900 times greater than that of $I_{dc}$, indicating the generation of a strong photocurrent (Iac).

On the other hand, other important parameters for determining the performance of the photoelectric device are detectivity (D) and responsivity (R).

Assuming that shot noise due to the dark current is the main cause of total noise, the detectivity (D) may be calculated using a following equation.

[Equation]

$$D = {R}/{(2qI_{dark})^{1/2}}$$

where R and q represent the responsivity and electronic charge, respectively.

On the other hand, the $R_{ac}$ and $D_{ac}$ values measured under the self-biased condition (0 V) achieve 8 mA W$^{-1}$ and 3.4 $\times$ 10$^{11}$ Jones at $\lambda$ = 620 nm, respectively. However, $R_{dc}$ may be as low as 0.91 mA W$^{-1}$.

(f) in FIG. 8 shows the wavelength-dependent behavior of $R_{ac}$ and $D_{ac}$. This superior detectivity allows for a higher signal level to yield a more accurate result.

<Characteristics of bio-sensing device including photoelectric device>

1. After passing the near-infrared pulsed light ($\lambda$ = 940 nm, 1 kHz, diameter = 6 mm) through the finger, the photocurrent output under the self-biased condition with or without external strain as applied was measured. The results are shown in (a) and (b) in FIG. 9.

Referring to (a) and (b) in FIG. 9, before applying the deformation, the biosensing device detected a relatively high first peak current ($I_{ac}$) of -3.0 $\mu$A, which corresponds to the "no damage" condition.

However, when the external strain was applied, the magnitude of the first peak current ($I_{ac}$) changed from -3.0 $\mu$A to -0.6 $\mu$A without changing the measurement condition (P = 3 mW cm$^{-2}$, 0 V, f = 1 kHz). This change is due to change in the density of the bio-material, and this difference in density reduces the intensity of the near-infrared ray passing through the finger. Thus, the damaged biological material may be detected.

Further, in (b) of FIG. 9, the response time of the bio-sensing device is smaller than about 7 ms. Thus, the device exhibits a fast detection response.

(c) in FIG. 9 is a graph showing the change in the photocurrent ($I_{ac}$) based on the applied external strain. (The applied external strain was calculated using a following equation based on change in a length (l) of the bio-material: Equation ($\Delta$l/l) $\times$ 100%, where $\Delta$l is the change in the length based on the applied strain.) As shown in (c) of FIG. 9, the change in the photocurrent based on the strain is linear. The linear slope of this graph is about 0.054, which represents the sensitivity (that is, current change/applied strain) of the biosensing device.

2. Since the biosensing device of the present disclosure has high sensitivity as described above, the device may detect small change in the biological material density, and thus may be used for body-strain mapping.

After scanning the finger as externally deformed by moving (1 mm/s) the infrared pulse generator, the photoelectric element, and the sensing element, a change value ($\Delta I_{ac}$) in the magnitude of the peak current measured by the sensing element was continuously mapped to obtain a one-to-one mapping image. (See FIG. 10)

Referring to (a) and (b) of FIG. 10, as may be seen in the original image (left) and the measured mapping image (right), the mapping images with/without the external strain applied thereto were clearly different and distinguishable from each other.

Specifically, $\Delta I_{ac}$ generated by the undeformed finger was about 2µA. $\Delta I_{ac}$ generated by the deformed finger was increased to about 3µA. As described above, the biosensing device of the present disclosure may generate the mapping image of the biological material based on the change in the magnitude of the peak current measured by the sensing element and may visualize the change in the density of the biological material based on the generated image.

Although the present disclosure has been described with reference to preferred embodiments, those skilled in the art will understand that various modifications and changes may be made to the present disclosure within the scope not departing from the spirit and scope of the present disclosure as described in the claims below.

Reference numerals

100: Infrared pulse generator 200: Photoelectric element
300: Sensing element

**Claims**

1. A bio-sensing device with a photoelectric element, the device comprising:

   an infrared pulse generator configured to irradiate infrared pulsed light to a target;
   the photoelectric element configured to receive the infrared pulsed light which has transmitted through the target, and to generate photocurrent based on the received light; and
   a sensing element configured to measure a magnitude of either a first peak current of the photocurrent corresponding to a leading edge of the infrared pulsed light or a second peak current of the photocurrent corresponding to a trailing edge of the infrared pulsed light, and to analyze the target based on the measurement result.

2. The bio-sensing device of claim 1, wherein a pulse period of the infrared pulsed light is in a range of 1 ms to 0.1 s,
   wherein a ratio of a pulse width to the pulse period of the infrared pulsed light is in a range of 1 to 10%.

3. The bio-sensing device of claim 1, wherein the photoelectric element includes:

   a p-type semiconductor layer;
   an n-type semiconductor layer formed on the p-type semiconductor layer such that a PN junction is formed between the p-type semiconductor layer and the n-type semiconductor layer, wherein the infrared pulsed light is incident on the n-type semiconductor layer; and
   a transparent current collector formed on a surface of the n-type semiconductor layer.

4. The bio-sensing device of claim 3, wherein the photoelectric element generates the photocurrent using a self-bias generated by the PN junction.

5. The bio-sensing device of claim 3, wherein the p-type semiconductor layer includes p-type silicon (p-Si),
   wherein the n-type semiconductor layer includes titanium dioxide ($TiO_2$).

6. The bio-sensing device of claim 3, wherein the transparent current collector includes at least one selected from silver nanowire, IGZO (Indium Gallium Zinc Oxide), IZO (Indium Zinc Oxide), SIZO (Silicon Indium Zinc Oxide), HIZO (Hafnium Indium Zinc Oxide), ZTO (Zinc Tin Oxide), ZnO, $Ga_2O_3$, $In_2O_3$, and $SnO_2$.

7. The bio-sensing device of claim 3, wherein the sensing element is electrically connected to and disposed between the p-type semiconductor layer and the n-type semiconductor layer.

8. The bio-sensing device of claim 1, wherein the bio-sensing device further comprises an image generator configured to generate a mapping image of the target based on change in a magnitude of the peak current measured by the sensing element.

9. A method for manufacturing a photoelectric element, the method comprising:

   depositing a titanium thin film on a substrate on which a p-type semiconductor layer has been formed in a sputtering process;
   oxidizing the titanium thin film to form a titanium dioxide ($TiO_2$) thin film; and
   forming a transparent current collector on a surface of the titanium dioxide ($TiO_2$) thin film.

**10.** The method of claim 9, wherein the titanium dioxide (TiO$_2$) thin film is formed to have a thickness of 10 to 100 nm.

**11.** The method of claim 9, wherein the p-type semiconductor layer includes p-type silicon (p-Si).

**12.** The method of claim 9, wherein the transparent current collector includes at least one selected from silver nanowire, IGZO (Indium Gallium Zinc Oxide), IZO (Indium Zinc Oxide), SIZO (Silicon Indium Zinc Oxide), HIZO (Hafnium Indium Zinc Oxide), ZTO (Zinc Tin Oxide), ZnO, Ga$_2$O$_3$, In$_2$O$_3$, and SnO$_2$.

FIG. 1

FIG. 2

FIG. 3

90% ... 90%

50% ... 50%

Pulse width

10% ... 10%

Leading edge duration ... Trailing edge duration

Period

DC photocurrent

(Second peak current)

AC photocurrent

(First peak current)

FIG. 4

FIG. 5

FIG. 6

FIG. 7

FIG. 8

FIG. 9

FIG. 10

**EP 4 289 345 A1**

**INTERNATIONAL SEARCH REPORT**

| International application No. |
| --- |
| **PCT/KR2022/000611** |

| | |
| --- | --- |
| **A.** | **CLASSIFICATION OF SUBJECT MATTER** |
| | **A61B 5/00**(2006.01)i; **H01L 31/0328**(2006.01)i; **H01L 31/18**(2006.01)i; **H01L 31/028**(2006.01)i; **H01L 31/032**(2006.01)i |

According to International Patent Classification (IPC) or to both national classification and IPC

| | |
| --- | --- |
| **B.** | **FIELDS SEARCHED** |

Minimum documentation searched (classification system followed by classification symbols)

A61B 5/00(2006.01); A61B 5/02(2006.01); C01G 23/04(2006.01); C23C 26/00(2006.01); G01J 1/02(2006.01); G01J 5/02(2006.01); G01N 21/27(2006.01); H01L 31/04(2006.01); H01L 31/0445(2014.01); H01L 51/44(2006.01); H01L 51/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 센서(sensor), 반도체(semiconductor), 이산화티타늄(TiO2), 적외(IR), 광전(optoelectronic), 생체(bio), 맵핑(mapping)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| Y | JP 2016-214512 A (TOSHIBA CORP.) 22 December 2016 (2016-12-22)<br>See paragraphs [0008]-[0018] and [0025]. | 1-7 |
| A | | 8-12 |
| Y | JP 2003-057110 A (MATSUSHITA ELECTRIC IND. CO., LTD.) 26 February 2003 (2003-02-26)<br>See paragraphs [0007] and [0061]. | 1-7 |
| Y | JP 6554300 B2 (KANEKA CORP.) 31 July 2019 (2019-07-31)<br>See paragraphs [0037], [0062] and [0095]; and figure 1. | 3-7,12 |
| Y | KR 10-2018-0013787 A (INDUSTRY-UNIVERSITY COOPERATION FOUNDATION HANYANG UNIVERSITY ERICA CAMPUS) 07 February 2018 (2018-02-07)<br>See claim 1; and paragraphs [0024], [0043] and [0084]. | 5,9-12 |

☑ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **12 May 2022** | **12 May 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**17**

EP 4 289 345 A1

**INTERNATIONAL SEARCH REPORT**

International application No.

**PCT/KR2022/000611**

| C. | DOCUMENTS CONSIDERED TO BE RELEVANT | |
|---|---|---|
| Category* | Citation of document, with indication, where appropriate, of the relevant passages | Relevant to claim No. |
| Y | JP 2002-121683 A (SEIKO EPSON CORP.) 26 April 2002 (2002-04-26)<br>See paragraphs [0061], [0066] and [0096]-[0106]. | 7,9-12 |

Form PCT/ISA/210 (second sheet) (July 2019)

18

| INTERNATIONAL SEARCH REPORT Information on patent family members | | | | International application No. PCT/KR2022/000611 | | |
|---|---|---|---|---|---|---|

| Patent document cited in search report | | | Publication date (day/month/year) | Patent family member(s) | | | Publication date (day/month/year) |
|---|---|---|---|---|---|---|---|
| JP | 2016-214512 | A | 22 December 2016 | CN | 106043997 | A | 26 October 2016 |
| | | | | US | 2016-0343775 | A1 | 24 November 2016 |
| | | | | US | 9812509 | B2 | 07 November 2017 |
| JP | 2003-057110 | A | 26 February 2003 | None | | | |
| JP | 6554300 | B2 | 31 July 2019 | JP | 2016-186968 | A | 27 October 2016 |
| KR | 10-2018-0013787 | A | 07 February 2018 | KR | 10-1939482 | B1 | 17 January 2019 |
| JP | 2002-121683 | A | 26 April 2002 | None | | | |

Form PCT/ISA/210 (patent family annex) (July 2019)